# EUROPEAN PATENT APPLICATION

(11) **EP 1 323 835 A2**
(43) Date of publication of application: **02.07.2003**
(21) Application number: 02028977.3
(22) Date of filing: 27.12.2002
(51) Int. Cl.: C12Q 1/68

(54) **Method for determining biospecies contained in test specimen and kit used for the same**

(30) Priority: 27.12.2001 JP 2001396943
(71) Applicant: Nisshinbo Industries, Inc., Tokyo (JP)
(72) Inventor: Yano, Hideo, Kyoto-shi, Kyoto (JP); Nishida, Michio, c/o System Research Incorporation, Tokyo (JP); Suzuki, Osamu, c/o Nisshinbo Industries, Inc., Chiba-shi, Chiba (JP)
(74) Representative: Tombling, Adrian George

(57) **Abstract**

In a method of determining presence or absence of a component derived from an organism contained in a test material or a biospecies from which the component is derived, a nucleic acid specific to a biospecies of interest is detected by performing hybridization of an oligonucleotide synthesized based on a sequence specific to the biospecies in a DNA sequence corresponding to a ribosomal RNA gene or a cytochrome-related gene of the biospecies of interest and a nucleic acid derived from the test material.

SEQUENCE LISTING

<110> Nisshinbo Industry Inc.

<120> METHOD FOR DETERMINING BIOSPECIES CONTAINED IN
      TEST SPECIMEN AND KIT USED FOR THE SAME

<130> OP1414

<140>
<141> 2002-12-27

<150> JP 2001-396943
<151> 2001-12-27

<160> 92

<170> PatentIn Ver. 3.0

<210> 1
<211> 16
<212> DNA
<213> Bos taurs

<220>
<223> capture

<400> 1
tcaccaattc ttgcta                                                    16

<210> 2
<211> 16
<212> DNA
<213> Bos taurs

<220>
<223> capture

<400> 2
gaaaaaaagt aagcgt                                                    16

<210> 3
<211> 16
<212> DNA
<213> Bos taurs

<220>
<223> capture

<400> 3
cctcaaatag attcag                                                    16

<210> 4
<211> 16
<212> DNA
<213> Bos taurs

<220>
<223> capture

<400> 4
atctaaccct atttaa                                                    16

<210> 5
<211> 16
<212> DNA
<213> Bos taurs

<400> 5
ttaaccatta aggaat                                                    16

<210> 6
<211> 16
<212> DNA
<213> Bos taurs

<220>
<223> capture

<400> 6
caacaatctc catgag                                                    16

<210> 7
<211> 16
<212> DNA
<213> Bos taurs

<400> 7
aggacatcct gatggt                                                    16

<210> 8
<211> 16
<212> DNA
<213> Bos taurs

<220>
<223> capture

<400> 8
tatctattac gtattt                                                    16

<210> 9
<211> 16
<212> DNA
<213> Bos taurs

<220>
<223> capture

<400> 9
ggtcttacac ttttct                                                    16

<210> 10
<211> 16
<212> DNA
<213> Bos taurs

<220>
<223> capture

<400> 10
tttatcatca tagcaa                                                    16

<210> 11
<211> 16
<212> DNA
<213> Bos taurs

<220>
<223> capture

<400> 11
atttcctcag acgtag                                                    16

<210> 12
<211> 16
<212> DNA
<213> Bos taurs

<220>
<223> capture

<400> 12
atctcctcag acgtag                                                    16

<210> 13
<211> 16
<212> DNA
<213> Gallus gallus

<220>
<223> capture

<400> 13
caaccacccc ttgcca                                                    16

<210> 14
<211> 16
<212> DNA
<213> Gallus gallus

<220>
<223> capture

<400> 14
gaacaacagt gagctc                                                    16

<210> 15
<211> 16
<212> DNA
<213> Gallus gallus

<220>
<223> capture

<400> 15
cttcacaagc catcaa                                                    16

<210> 16
<211> 16
<212> DNA
<213> Gallus gallus

<220>
<223> capture

<400> 16
caataaatat atactt                                                    16

<210> 17
<211> 15
<212> DNA
<213> Gallus gallus

<220>
<223> capture

<400> 17
agccccactg ggtcc                                                     15

<210> 18

<211> 16
<212> DNA
<213> Gallus gallus

<220>
<223> capture

<400> 18
ccacacataa acccct                                                    16

<210> 19
<211> 16
<212> DNA
<213> Gallus gallus

<220>
<223> capture

<400> 19
aggacaacct aatggt                                                    16

<210> 20
<211> 16
<212> DNA
<213> Gallus gallus

<220>
<223> capture

<400> 20
tatctatgga cactcc                                                    16

<210> 21
<211> 16
<212> DNA
<213> Gallus gallus

<220>
<223> capture

<400> 21

gctcctacct ctacaa 16

<210> 22
<211> 16
<212> DNA
<213> Gallus gallus

<220>
<223> capture

<400> 22
ttgcaatcgc aggtat 16

<210> 23
<211> 16
<212> DNA
<213> Gallus gallus

<220>
<223> capture

<400> 23
atctcatccg actctg 16

<210> 24
<211> 16
<212> DNA
<213> Ovis aries

<220>
<223> capture

<400> 24
tcaccaatcc ttgcta 16

<210> 25
<211> 16
<212> DNA
<213> Ovis aries

<220>

<223> capture

<400> 25
ggacaaaagt aagctc                                                    16

<210> 26
<211> 16
<212> DNA
<213> Ovis aries

<220>
<223> capture

<400> 26
cctcaagtaa atatga                                                    16

<210> 27
<211> 16
<212> DNA
<213> Ovis aries

<220>
<223> capture

<400> 27
acttaaacct atttac                                                    16

<210> 28
<211> 16
<212> DNA
<213> Ovis aries

<220>
<223> capture

<400> 28
   tcaaccacca agggat                                                     16

<210> 29
<211> 16
<212> DNA

<213> Ovis aries

<220>
<223> capture

<400> 29
caacactcct tatgag                                                    16

<210> 30
<211> 16
<212> DNA
<213> Ovis aries

<220>
<223> capture

<400> 30
aggacacccc gatggt                                                    16

<210> 31
<211> 16
<212> DNA
<213> Ovis aries

<220>
<223> capture

<400> 31
tatctgttat gtattt                                                    16

<210> 32
<211> 16
<212> DNA
<213> Ovis aries

<400> 32
gatcatatac cttcct                                                    16

<210> 33
<211> 16
<212> DNA

<213> Ovis aries

<220>
<223> capture

<400> 33
ttcatcatcg cagccc                                                    16

<210> 34
<211> 16
<212> DNA
<213> Ovis aries

<220>
<223> capture

<400> 34
ttcatcatcg caggcc                                                    16

<210> 35
<211> 16
<212> DNA
<213> Ovis aries

<220>
<223> capture

<400> 35
attccatcgg acacag                                                    16

<210> 36
<211> 16
<212> DNA
<213> Sus scrofa

<220>
<223> capture

<400> 36
ttaccaaccc ttgcca                                                    16

<210> 37
<211> 16
<212> DNA
<213> Sus scrofa

<220>
<223> capture

<400> 37
gaacaatagt aagcac                                                    16

<210> 38
<211> 16
<212> DNA
<213> Sus scrofa

<220>
<223> capture

<400> 38
cctcaagcat gtagta                                                    16

<210> 39
<211> 16
<212> DNA
<213> Sus scrofa

<220>
<223> capture

<400> 39
aaaaataacc tatatt                                                    16

<210> 40
<211> 16
<212> DNA
<213> Sus scrofa

<220>
<223> capture

<400> 40
tcaaccacaa agggat                                                    16

<210> 41
<211> 16
<212> DNA
<213> Sus scrofa

<220>
<223> capture

<400> 41
aacataactt aacatg                                                    16

<210> 42
<211> 16
<212> DNA
<213> Sus scrofa

<220>
<223> capture

<400> 42
aggacaccca aatggt                                                    16

<210> 43
<211> 16
<212> DNA
<213> Sus scrofa

<220>
<223> capture

<400> 43
tatctattat aaattt                                                    16

<210> 44
<211> 16
<212> DNA
<213> Sus scrofa

<220>
<223> capture

<400> 44
gatcctatat attcct                                                    16

<210> 45
<211> 16
<212> DNA
<213> Sus scrofa

<220>
<223> capture

<400> 45
ttcatcatta ccgccc                                                    16

<210> 46
<211> 16
<212> DNA
<213> Sus scrofa

<220>
<223> capture

<400> 46
atctcatcag acatag                                                    16

<210> 47
<211> 16
<212> DNA
<213> Equus caballus

<220>
<223> capture

<400> 47
ccaccatccc ttgcta                                                    16

<210> 48
<211> 16

<212> DNA
<213> Equus caballus

<220>
<223> capture

<400> 48
gtaccgaagt aagcac                                                        16

<210> 49
<211> 16
<212> DNA
<213> Equus caballus

<220>
<223> capture

<400> 49
ccttaaatat cacaaa                                                        16

<210> 50
<211> 16
<212> DNA
<213> Equus caballus

<220>
<223> capture

<400> 50
aacataacat aaaacc                                                        16

<210> 51
<211> 15
<212> DNA
<213> Equus caballus

<220>
<223> capture

<400> 51
ttaaccttca gggac                                                         15

<210> 52
<211> 16
<212> DNA
<213> Equus caballus

<220>
<223> capture

<400> 52
caaaactttt gattga                                                    16

<210> 53
<211> 16
<212> DNA
<213> Equus caballus

<220>
<223> capture

<400> 53
aagacatcct aatggt                                                    16

<210> 54
<211> 16
<212> DNA
<213> Equus caballus

<220>
<223> capture

<400> 54
tatctattct atactt                                                    16

<210> 55
<211> 16
<212> DNA
<213> Equus caballus

<220>
<223> capture

<400> 55
gctcttacac attcct                                                    16

<210> 56
<211> 16
<212> DNA
<213> Equus caballus

<220>
<223> capture

<400> 56
ttcatcatca cagccc                                                    16

<210> 57
<211> 16
<212> DNA
<213> Equus caballus

<220>
<223> capture

<400> 57
atcccatccg atatgg                                                    16

<210> 58
<211> 18
<212> DNA
<213> Artificial/Unknown

<220>
<223> primer: amplify 12S rRNA gene

<400> 58
aaaactcaaa ggacttgg                                                  18

<210> 59
<211> 18
<212> DNA
<213> Artificial/Unknown

<220>
<223> primer: amplify 12S rRNA gene

<400> 59
aaaactctaa ggacttgg                                                    18

<210> 60
<211> 18
<212> DNA
<213> Artificial/Unknown

<220>
<223> primer: amplify 12S rRNA gene

<400> 60
gaaactcaaa ggacttgg                                                    18

<210> 61
<211> 18
<212> DNA
<213> Artificial/Unknown

<220>
<223> primer: amplify 12S rRNA gene

<400> 61
agagaatgta gcccattt                                                    18

<210> 62
<211> 18
<212> DNA
<213> Artificial/Unknown

<220>
<223> primer: amplify 12S rRNA gene

<400> 62
agaaaatgta gcccattt                                                    18

<210> 63

<211> 17
<212> DNA
<213> Artificial/Unknown

<220>
<223> primer: amplify 12S rRNA gene

<400> 63
aggaggattt agcagta                                                    17

<210> 64
<211> 18
<212> DNA
<213> primer: amplify 12S rRNA gene

<220>
<223> capture

<400> 64
ttaccttgtt acgacttg                                                   18

<210> 65
<211> 18
<212> DNA
<213> Artificial/Unknown

<220>
<223> primer: amplify 16S rRNA gene

<400> 65
aagacgagaa gaccctat                                                   18

<210> 66
<211> 18
<212> DNA
<213> Artificial/Unknown

<220>
<223> primer: amplify 16S rRNA gene

<400> 66

aagacgagaa gaccctgt 18

<210> 67
<211> 15
<212> DNA
<213> Artificial/Unknown

<220>
<223> primer: amplify 16S rRNA gene

<400> 67
tgcgctgtta tccct 15

<210> 68
<211> 16
<212> DNA
<213> Artificial/Unknown

<220>
<223> primer: amplify 16S rRNA gene

<400> 68
acgacctcga tgttgg 16

<210> 69
<211> 18
<212> DNA
<213> Artificial/Unknown

<220>
<223> primer: amplify 16S rRNA gene

<400> 69
tgtcctttcg tactggga 18

<210> 70
<211> 18
<212> DNA
<213> Artificial/Unknown

<220>

<223> primer: amplify 16S rRNA gene

<400> 70
ggtcctttcg tactagga                                                    18

<210> 71
<211> 17
<212> DNA
<213> Artificial/Unknown

<220>
<223> primer: amplify cytochrome b gene

<400> 71
gatacataca cgcaaac                                                     17

<210> 72
<211> 17
<212> DNA
<213> Artificial/Unknown

<220>
<223> primer: amplify cytochrome b gene

<400> 72
ggaatctcca cgcaaac                                                     17

<210> 73
<211> 16
<212> DNA
<213> Artificial/Unknown

<220>
<223> primer: amplify cytochrome b gene

<400> 73
ctacctccat gccaac                                                      16

<210> 74
<211> 17
<212> DNA

<213> Artificial/Unknown

<220>
<223> primer: amplify cytochrome b gene

<400> 74
gctatctaca tgcaaac                                                    17

<210> 75
<211> 18
<212> DNA
<213> Artificial/Unknown

<220>
<223> primer: amplify cytochrome b gene

<400> 75
cgatatatac acgcaaac                                                   18

<210> 76
<211> 18
<212> DNA
<213> Artificial/Unknown

<220>
<223> primer: amplify cytochrome b gene

<400> 76
agaatgatat ttgtcctc                                                   18

<210> 77
<211> 18
<212> DNA
<213> Artificial/Unknown

<220>
<223> primer: amplify cytochrome b gene

<400> 77
agaatgatat ttggcccc                                                   18

<210> 78
<211> 18
<212> DNA
<213> Artificial/Unknown

<220>
<223> primer: amplify cytochrome b gene

<400> 78
aaaaggatat ttggcctc                                                    18

<210> 79
<211> 16
<212> DNA
<213> Artificial/Unknown

<220>
<223> primer: amplify cytochrome b gene

<400> 79
caacccttac ccgatt                                                      16

<210> 80
<211> 16
<212> DNA
<213> Artificial/Unknown

<220>
<223> primer: amplify cytochrome b gene

<400> 80
ccacccttac ccgatt                                                      16

<210> 81
<211> 16
<212> DNA
<213> Artificial/Unknown

<220>
<223> primer: amplify cytochrome b gene

<400> 81
caaccctcac acgatt                                                    16

<210> 82
<211> 16
<212> DNA
<213> Artificial/Unknown

<220>
<223> primer: amplify cytochrome b gene

<400> 82
ctaccctcac ccgatt                                                    16

<210> 83
<211> 17
<212> DNA
<213> Artificial/Unknown

<220>
<223> primer: amplify cytochrome b gene

<400> 83
gggtggaatg ggatttt                                                   17

<210> 84
<211> 17
<212> DNA
<213> Artificial/Unknown

<220>
<223> primer: amplify cytochrome b gene

<400> 84
gggtgaaatg gaatttt                                                   17

<210> 85
<211> 17
<212> DNA
<213> Artificial/Unknown

<220>
<223> primer: amplify cytochrome b gene

<400> 85
gggtggaagg gaatttt                                                    17

<210> 86
<211> 18
<212> DNA
<213> Artificial/Unknown

<220>
<223> capture

<400> 86
cctgtcacga gtggatta                                                   18

<210> 87
<211> 96
<212> DNA
<213> Artificial/Unknown

<220>
<223> nucleotide probe

<400> 87
aaaactcaaa ggacttggcc tgtcctgcag gcctcccctc ctgtcacgag tggattaacg 60
gctgggaaga gttgaccaaa atgggctaca ttctct                               96

<210> 88
<211> 96
<212> DNA
<213> Artificial/Unknown

<220>
<223> nucleotide probe

<400> 88
agagaatgta gcccattttg gtcaactctt cccagccgtt aatccactcg tgacaggagg 60
ggaggcctgc aggacaggcc aagtcctttg agtttt                               96

<210> 89
<211> 93
<212> DNA
<213> Artificial/Unknown

<220>
<223> nucleotide probe

<400> 89
aagacgagaa gaccctatcc tgtcctgcag gcctcccctc ctgtcacgag tggattaacg 60
gctgggaaga gttgaccaag ggataacagc gca 93

<210> 90
<211> 93
<212> DNA
<213> Artificial/Unknown

<220>
<223> nucleotide probe

<400> 90
tgcgctgtta tcccttggtc aactcttccc agccgttaat ccactcgtga caggagggga 60
ggcctgcagg acaggatagg gtcttctcgt ctt 93

<210> 91
<211> 95
<212> DNA
<213> Artificial/Unknown

<220>
<223> nucleotide probe

<400> 91
gatacataca cgcaaaccct gtcctgcagg cctcccctcc tgtcacgagt ggattaacgg 60
ctgggaagag ttgaccagag gacaaatatc attct 95

<210> 92
<211> 95
<212> DNA
<213> Artificial/Unknown

<220>
<223> nucleotide probe

<400> 92
agaatgatat ttgtcctctg gtcaactctt cccagccgtt aatccactcg tgacaggagg 60
ggaggcctgc aggacagggt ttgcgtgtat gtatc 95

## Description

### Background of the Invention

### Field of the Invention

The present invention relates to a method and kit for determining a biospecies contained in a test specimen. More precisely, the present invention relates to a method for determining whether a tissue or cell component derived from a specific biospecies is contained in a test material such as feed, feed raw material, feed additive, feed-related product, processed food, processed food raw material, food additive, pharmaceutical preparation, pharmaceutical additive, functional food, cosmetic or cosmetic additive, and an extremely accurate and highly operable determination kit used for the same. The present invention further relates to a capture oligonucleotide and a DNA probe used in this kit and methods for producing the same.

### Description of the Related Art

The most intensively studied method for examining a specific biospecies contained in a test material is a method of excising a specific genetic region and determining its sequence. This method is intended to identify a gene sequence unique to a biospecies. A gene sequence (DNA or RNA) of interest is amplified, and its existence is determined by using an oligonucleotide probe. Examples thereof include the PCR method (polymerase chain reaction, Roche), an analogous method, the MTD method (using reverse transcriptase and RNA polymerase, Chugai Pharmaceutical), and so forth. Several gene sequence identification methods have been developed by applying these fundamental techniques.

To confirm existence of a specific gene, there are generally used a method of determining a nucleic acid sequence (sequencing) derived from a test material, a method of analyzing an electrophoretic pattern of a gene amplification product, a method of identifying existence of a target gene sequence by separating fragments of a gene amplification product digested with restriction enzymes by electrophoresis and classifying the obtained electrophoretic patterns and so forth.

### 1. Background of need for the present invention

Demands for identification of a tissue or cell component derived from a biospecies contained in a test material are rapidly increasing. While such identification has traditionally meant detection of virus or animal contamination, detection of those other than pathogens also attracts attention recently. Demands for testing are rapidly increasing against the background of social unrests such as the problem of labeling of genetically manipulated maize and soybean, the problem of contamination of feed with meat and bone meal and restriction on animal-derived raw materials for pharmaceutical preparations and cosmetics.

In particular, in conjunction with the problem of bovine spongiform encephalopathy, contamination of cattle feed and feed additives with meat and bone meal has become a social problem. Establishment of techniques to guarantee non-contamination of ruminant components in various feed-related products and imported feed materials is desired as the most effective protective means against occurrence of these problems.

### 2. Inadequacies in current techniques

Although the conventional techniques mentioned above enable detection of contamination with or inclusion of a tissue or cell component derived from a specific biospecies in a test material, their test procedures are complicated, and test require techniques of a certain level. Therefore, they need to be improved for common use. Targeted improvements include improvement of precision and operatability of tests and reduction of testing costs.

The most common method for confirming existence of a biospecies is determination of a gene sequence (sequencing). Specifically, a nucleotide sequence is determined by incorporating dideoxynucleotides when a strand complementary to DNA serving as a template is synthesized to obtain complementary strands in various lengths and separating them by electrophoresis. Although automatic sequencers automating this method are commercially available, these are currently still expensive and therefore not widely used in general tests. Further, sample preparation operation is complicated since purification is required after extraction of nucleic acids from a sample, for example. Further, since the sequence must be read from an electrophoresis image of each sample after an enzymatic reaction, this method is not suitable for a general test for manipulating a large number of specimens.

As a method for confirming existence of a specific gene, a method of analyzing an electrophoretic pattern of a gene amplification product is also used. A nucleotide sequence that universally exists in biospecies and has a sequence specific to each biospecies is utilized to amplify a gene by using the biospecies-specific sequences as primers. Then, the obtained amplification product is analyzed based on the electrophoresis pattern thereof. This method is characterized in that, at this time, the primers are designed so that the amplification products should have different lengths. By analyzing sizes of bands observed in electrophoresis, genes existing in the specimen can be elucidated.

In the aforementioned method, the primers are designed for each biospecies. When amplification is separately carried out by using each set of primers, a large number of tubes are required, and hence operations become extremely complicated. Even if amplification is carried out in one tube by mixing all the primers, more strict temperature control is required for accurate annealing reactions of the primers to template DNA. Further, a gel medium often used for the electrophoresis of the amplification products, such as acrylamide, has disadvantages that discrimination of a small difference in molecular weights is difficult, that errors occur due to non-uniformity of the gel medium and so forth.

Further, as a critical defect of this method, errors attributable to the analysis of the strand lengths alone can be mentioned. In the amplification reactions, impurities in a sample are often preferentially amplified, and this may result in determination errors when a strand length equal to an expected strand length is observed.

To make up for the aforementioned defects, there is a method of separating fragments of a gene amplification product obtained by digesting the amplification product with restriction enzymes by electrophoresis and classifying electrophoretic patterns to confirm the existence of a target gene sequence. As described above, a primer is designed so as to include a biospecies-specific sequence by utilizing a nucleotide sequence of a gene that universally exists in each biospecies and has a biospecies-specific sequence, and after amplification, the amplification product is digested with a restriction enzyme that recognizes the specific sequence. Then, the products are separated by electrophoresis and the biospecies having the gene is identified based on the obtained band size.

This method also has problems. Since each biospecies-specific sequence is recognized by a different restriction enzyme, the same number of restriction enzyme recognition sites as the number of biospecies must be identified. However, the same number of sites do not necessarily exist. Further, even when they exist, the obtained amplification product must be digested with several types of restriction enzymes and subjected to electrophoresis, and this is an extremely complicated operation. Further, the aforementioned problems of the electrophoresis are not solved.

### Summary of the Invention

The present invention was accomplished in view of the aforementioned problems, and an object of the present invention is to provide a method enabling accurate determination for whether a tissues or cell component derived from a specific biospecies is contained in a sample such as feed, feed additive, feed-related product, processed food, processed food raw material, food additive, pharmaceutical preparation, pharmaceutical additive, functional food, cosmetic or cosmetic additive.

The inventors of the present invention has been developed studies to achieve the aforementioned object. As a result, they found that a biospecies could be accurately and efficiently determined by hybridization using an oligonucleotide synthesized based on a sequence of a ribosomal RNA gene or a cytochrome-related gene specific to the biospecies, and thus accomplished the present invention.

That is, the present invention provides the followings.
(1) A method for determining presence or absence of a component derived from an organism contained in a test material or a biospecies from which the component is derived, which comprises detecting a nucleic acid specific to a biospecies of interest by performing hybridization of an oligonucleotide synthesized based on a sequence specific to the biospecies in a DNA sequence corresponding to a ribosomal RNA gene or a cytochrome-related gene of the biospecies of interest and a nucleic acid derived from the test material.
(2) The method according to (1), wherein the biospecies is one or more kinds selected from bovine, ovine, swine, equine and fowl.
(3) The method according to (1), wherein the test material is feed, feed raw material, feed additive, feed-related product, processed food, processed food raw material, food additive, pharmaceutical preparation, pharmaceutical additive, functional food, cosmetic or cosmetic additive.
(4) The method according to any one of (1) to (3), wherein the DNA sequence corresponding to a ribosomal RNA gene is a DNA sequence corresponding to the 12S ribosomal RNA gene or the 16S ribosomal RNA gene, and the DNA sequence corresponding to a cytochrome-related gene is a DNA sequence corresponding to the cytochrome b gene.
(5) The method according to any one of (1) to (4), wherein the biospecies is bovine, the DNA sequence corresponding to the 12S ribosomal RNA gene contains any of the nucleotide sequences of SEQ ID NOS: 1 to 4, the DNA sequence corresponding to the 16S ribosomal RNA gene contains any of the nucleotide sequences of SEQ ID NOS: 5 to 8, and the DNA sequence corresponding to the cytochrome b gene contains any of the nucleotide sequences of SEQ ID NOS: 9 to 12.
(6) The method according to any one of (1) to (4), wherein the biospecies is fowl, the DNA sequence corresponding to the 12S ribosomal RNA gene contains any of the nucleotide sequences of SEQ ID NOS: 13 to 16, the DNA sequence corresponding to the 16S ribosomal RNA gene contains any of the nucleotide sequences of SEQ ID NOS: 17 to 20, and the DNA sequence corresponding to the cytochrome b gene contains any of the nucleotide sequences of SEQ ID NOS: 21 to 23.
(7) The method according to any one of (1) to (4), wherein the biospecies is ovine, the DNA sequence corresponding to the 12S ribosomal RNA gene contains any of the nucleotide sequences of SEQ ID NOS: 24 to 27, the DNA sequence corresponding to the 16S ribosomal RNA gene contains any of the nucleotide sequences of SEQ ID NOS: 28 to 31, and the DNA sequence corresponding to the cytochrome b gene contains any of the nucleotide sequences of SEQ ID NOS: 32 to 35.
(8) The method according to any one of (1) to (4), wherein the biospecies is swine, the DNA sequence corresponding to the 12S ribosomal RNA gene contains any of the nucleotide sequences of SEQ ID NOS: 36 to 39, the DNA sequence corresponding to the 16S ribosomal RNA gene contains any of the nucleotide sequences of SEQ ID NOS: 40 to 43, and the DNA sequence corresponding to the cytochrome b gene contains any of the nucleotide sequences of SEQ ID NOS: 44 to 46.
(9) The method according to any one of (1) to (4), wherein the biospecies is equine, the DNA sequence corresponding to the 12S ribosomal RNA gene contains any of the nucleotide sequences of SEQ ID NOS: 47 to 50, the DNA sequence corresponding to the 16S ribosomal RNA gene contains any of the nucleotide sequences of SEQ ID NOS: 51 to 54, and the DNA sequence corresponding to the cytochrome b gene contains any of the nucleotide sequences of SEQ ID NOS: 55 to 57.
(10) A kit for determining presence or absence of a component derived from an organism contained in a test material or a biospecies from which the component is derived, which comprises:
   a substrate on which one or more oligonucleotide synthesized based on a sequence specific to a biospecies of interest in a DNA sequence corresponding to a ribosomal RNA gene or a cytochrome-related gene of the biospecies are immobilized with a covalent bond, wherein
   a nucleic acid specific to the biospecies of interest is detected by hybridization of the oligonucleotide and a nucleic acid derived from the test material.
(11) The kit according to (10), wherein a carbodiimide group or an isocyanate group is provided on the surface of the substrate so that the covalent bond should be formed by a reaction of the carbodiimide group or isocyanate group and the oligonucleotide or a linker added to an end of the oligonucleotide.
(12) The kit according to (10), wherein the biospecies is one or more kinds selected from bovine, ovine, swine, equine and fowl.
(13) The kit according to (10), wherein the test material is feed, feed raw material, feed additive, feed-related product, processed food, processed food raw material, food additive, pharmaceutical preparation, pharmaceutical additive, functional food, cosmetic or cosmetic additive.
(14) The kit according to any one of (10) to (13), wherein the DNA sequence corresponding to a ribosomal RNA gene is a DNA sequence corresponding to the 12S ribosomal RNA gene or the 16S ribosomal RNA gene, and the DNA sequence corresponding to a cytochrome-related gene is a DNA sequence corresponding to the cytochrome b gene.
(15) The kit according to any one of (10) to (14), wherein the biospecies is bovine, the DNA sequence corresponding to the 12S ribosomal RNA gene contains any of the nucleotide sequences of SEQ ID NOS: 1 to 4, the DNA sequence corresponding to the 16S ribosomal RNA gene contains any of the nucleotide sequences of SEQ ID NOS: 5 to 8, and the DNA sequence corresponding to the cytochrome b gene contains any of the nucleotide sequences of SEQ ID NOS: 9 to 12.
(16) The kit according to any of (10) to (14), wherein the biospecies is fowl, the DNA sequence corresponding to the 12S ribosomal RNA gene contains any of the nucleotide sequences of SEQ ID NOS: 13 to 16, the DNA sequence corresponding to the 16S ribosomal RNA gene contains any of the nucleotide sequences of SEQ ID NOS: 17 to 20, and the DNA sequence corresponding to the cytochrome b gene contains any of the nucleotide sequences of SEQ ID NOS: 21 to 23.
(17) The kit according to any of (10) to (14), wherein the biospecies is ovine, the DNA sequence corresponding to the 12S ribosomal RNA gene contains any of the nucleotide sequences of SEQ ID NOS: 24 to 27, the DNA sequence corresponding to the 16S ribosomal RNA gene contains any of the nucleotide sequences of SEQ ID NOS: 28 to 31, and the DNA sequence corresponding to the cytochrome b gene contains any of the nucleotide sequences of SEQ ID NOS: 32 to 35.
(18) The kit according to any of (10) to (14), wherein the biospecies is swine, the DNA sequence corresponding to the 12S ribosomal RNA gene contains any of the nucleotide sequences of SEQ ID NOS: 36 to 39, the DNA sequence corresponding to the 16S ribosomal RNA gene contains any of the nucleotide sequences of SEQ ID NOS: 40 to 43, and the DNA sequence corresponding to the cytochrome b gene contains any of the nucleotide sequences of SEQ ID NOS: 44 to 46.
(19) The kit according to any of (10) to (14), wherein the biospecies is equine, the DNA sequence corresponding to the 12S ribosomal RNA gene contains any of the nucleotide sequences of SEQ ID NOS: 47 to 50, the DNA sequence corresponding to the 16S ribosomal RNA gene contains any of the nucleotide sequences of SEQ ID NOS: 51 to 54, and the DNA sequence corresponding to the cytochrome b gene contains any of the nucleotide sequences of SEQ ID NOS: 55 to 57.
(20) A method for producing a labeled DNA probe derived from DNA of a test material used for identifying a biospecies by using the method according to (1), comprising the steps of:
   extracting DNA from the test material, and
   performing DNA amplification by using the DNA as a template and a primer of which 5' or 3' end is labeled with a fluorescent substance or a hapten.
(21) A DNA probe obtainable by the method according to (20), which is labeled with any of fluorescent substances including fluorescein (FITC), Rhodamine, phycoerythrin (PE), Texas Red and cyanine fluorochrome.
(22) A DNA probe obtainable by the method according to (20), which is labeled with any of haptens including biotin, digoxigenin (Dig), dinitrophenyl (DNP) and fluorescein.
(23) A method for producing a labeled DNA probe derived from DNA of a test material used for identifying a biospecies by using the kit according to (10), comprising the steps of:
   extracting DNA from the test material, and
   performing DNA amplification by using the DNA as a template and a primer of which 5' or 3' end is labeled with a fluorescent substance or a hapten.
(24) A primer for producing a nucleic acid derived from DNA of a test material used in the method according to (1), which has a sequence corresponding to the 12S ribosomal RNA gene of any one or more kinds of animals selected from bovine, ovine, equine, swine and fowl and has a nucleotide sequence selected from the nucleotide sequences of SEQ ID NOS: 58 to 64.
(25) A primer for producing a nucleic acid derived from DNA of a test material used in the method according to (1), which has a sequence corresponding to the 16S ribosomal RNA gene of any one or more kinds of animals selected from bovine, ovine, equine, swine and fowl and has a nucleotide sequence selected from the nucleotide sequences of SEQ ID NOS: 65 to 70.
(26) A primer for producing a nucleic acid derived from DNA of a test material used in the method according to (1), which has a sequence corresponding to the cytochrome b gene of any one or more kinds of animals selected from bovine, ovine, equine, swine and fowl and has a nucleotide sequence selected from the nucleotide sequences of SEQ ID NOS: 71 to 85.
(27) The method according to any one of (1) to (9), wherein, when the oligonucleotide has a hairpin structure, loop structure or another three-dimensional structure that interferes with hybridization with the test nucleic acid, there is used an oligonucleotide corresponding to the oligonucleotide of which three-dimensional structure is canceled by substitution of inosine or a nucleic acid that does not pair with any nucleotide for one or more nucleotides constituting the oligonucleotide.
(28) The kit according to (10) to (19), wherein, when the oligonucleotide has a hairpin structure, loop structure or another three-dimensional structure that interferes with hybridization with the test nucleic acid, there is used an oligonucleotide corresponding to the oligonucleotide of which three-dimensional structure is canceled by substitution of inosine or a nucleic acid that does not pair with any nucleotide for one or more nucleotides constituting the oligonucleotide.
(29) The kit according to (11), wherein the reaction of the carbodiimide group or isocyanate group on the substrate and the oligonucleotide is carried out under ultraviolet ray irradiation.

In the present invention, a biospecies mainly refers to an academic classification, but identification of a variety or a transgenic organism is also encompassed in a scope to which the present invention can be applied.

The present invention provides a method enabling identification of tissues or cell components derived from various biospecies. As for the use thereof, the method of the present invention can be used to detect contamination of feed or products for rearing, processed food, processed food raw material, food additive, pharmaceutical preparation or cosmetic with components derived from an animal. Hereafter, as a specific example, a method and kit for determining whether feed is contaminated with a raw material derived from an animal will be described.

An animal biospecies of which component contained in a test material is determined by using one or more of DNA sequences corresponding to ribosomal RNA or DNA sequences or corresponding to cytochrome-related genes specific to any one or more kinds of bovine (scientific name: *Bos taurus*), ovine (scientific name: *Ovis aries*), swine (scientific name: *Sus scrofa*), equine (scientific name: *Equus caballus*) and fowl (scientific name: *Gallus gallus*). The animal biospecies of which component is contained in the test material is determined by preparing an oligonucleotide designed based on a sequence specific to each animal biospecies and hybridizing it with a nucleic acid that is extracted from the test material and amplified. As a method used for this test, for example, the hybridization can be performed on a membrane, but a DNA array type kit has been developed as the most effective method. This kit comprises a substrate on which one or more oligonucleotides designed and synthesized based on the aforementioned DNA sequence are immobilized by using the method developed by the inventors of the present invention, and is used to identify content of DNA derived from any one or more kinds of bovine, ovine, swine, equine and fowl by hybridization with a nucleic acid extracted from a test material. More specifically, a DNA sequence corresponding to the 12S ribosomal RNA (hereinafter referred to as 12S rRNA) or the 16S ribosomal RNA (hereinafter referred to as 16S rRNA) is used as the ribosomal RNA, and the cytochrome b sequence is used as the cytochrome-related gene sequence.

The oligonucleotide provided by the present invention is derived from a biospecies-specific sequence region on 12S rRNA, 16S rRNA or cytochrome b of bovine or the like, and it is designed and synthesized to have a length of 12 to 30 nucleotides. Further, the present invention also provides an oligonucleotide that has a nucleotide sequence obtained by extending or shortening a gene sequence of the characteristic sequence of which position on the genome matches in bovine and other animals for the 5' or 3' side or the both of a nucleotide sequence of a gene from which the oligonucleotide is derived by using each nucleotide sequence as a basic sequence, in which the characteristic site contained in the original nucleotide sequence is not include substitution.

The kit of the present invention is characterized by containing the following (a), (b), (c) or a combination thereof:
(a) a sequence that is derived from the 12S rRNA gene of bovine, ovine, equine, swine or fowl and designed to have 12 to 24 nucleotides, and typical examples thereof include the nucleotide sequences of SEQ ID NOS: 1 to 4, 13 to 16, 24 to 27, 36 to 39 and 47 to 50, any of which can be used solely or in combination of two or more of them;
(b) a sequence that is derived from the 16S rRNA gene of bovine, ovine, equine, swine or fowl and designed to have 12 to 24 nucleotides, and typical examples thereof include the nucleotide sequences of SEQ ID NOS: 5 to 8, 17 to 20, 28 to 31, 40 to 43 and 51 to 54, any of which can be used solely or in combination of two or more of them; and
(c) a sequence that is derived from the cytochrome b gene of bovine, ovine, equine, swine or fowl and designed to have 12 to 24 nucleotides, and typical examples thereof include the nucleotide sequences of SEQ ID NOS: 9 to 12, 21 to 23, 32 to 35, 44 to 46 and 55 to 57, any of which can be used solely or in combination of two or more of them.
   According to the present invention, an artificially designed positive control and a negative control corresponding thereto can be further incorporated into the determination kit in addition to a synthetic oligonucleotide prepared based on a gene sequence of each animal biospecies. Two kinds of cases are conceived for a method for preparing a positive control. One is a case where a part of a nucleotide sequence that surely exists in a test specimen can be used, and the other is a case where an appropriate nucleotide sequence derived from an animal may not exist like feed that does not contain an animal material.
   The positive control for the former case is used to confirm a positive reaction of hybridization of primer DNA derived from the test specimen and a capture oligo as a marker and is:
(d) a synthetic oligonucleotide designed by selecting any animal biospecies contained in the test specimen and excising a part of its gene in an appropriate length like a capture oligo.
   The negative control corresponding thereto is:
(e) a synthetic oligonucleotide designed as a nucleotide sequence corresponding to the nucleotide sequence of the oligonucleotide of the aforementioned (d) including substitution for one or more nucleotides in the range of less than 20% of the length of the nucleotide sequence.
   The positive control for the latter case is incorporated into the kit in order to avoid erroneous determination due to a failure in test operations when it is more emphasized to ensure that the test procedures from amplification of the test specimen DNA using the primer to the hybridization with the capture oligo are correctly carried out, and is:
(f) a synthetic oligonucleotide artificially produced based on an assumption of a nucleotide sequence that does not naturally exist as a test specimen or a gene of an animal biospecies as a test subject, which is confirmed as a result of search of a gene database, and does not match hybridization conditions with the artificially produced test gene. One example thereof is shown as SEQ ID NOS: 86.
   The negative control corresponding thereto is:
(g) a synthetic oligonucleotide designed as a nucleotide sequence corresponding to the nucleotide sequence of the oligonucleotide of the aforementioned (f) including substitution for one or more nucleotides in the range of less than 20% of the length of the nucleotide sequence.

Further, the present invention provides a method for obtaining a labeled DNA probe derived from a nucleic acid of a test animal for determining one or more kinds of any of bovine, ovine, equine, swine and fowl.

That is, a DNA probe labeled with a fluorescent substance is provided by extracting DNA from feed; and

Performing nucleic acid amplification by using this DNA as a template and a labeled primer with its 5' or 3' end labeled with a fluorescent substance or a hapten.

The present invention also provides:
a primer set for amplifying the 12S rRNA gene that is an oligonucleotide pair having nucleotide sequences selected from those of SEQ ID NOS: 58 to 64, and uses one or more combinations of a mixture of SEQ ID NOS: 58 to 60 and a mixture of SEQ ID NOS: 61 and 62, or SEQ ID NO: 63 and SEQ ID NO: 64;
a primer set for amplifying the 16S rRNA gene that is an oligonucleotide pair having nucleotide sequences selected from those of SEQ ID NOS: 65 to 70, and uses one or more combinations of a mixture of SEQ ID NOS: 65 and 66 and SEQ ID NO: 67, or SEQ ID NO: 68 and a mixture of SEQ ID NOS: 69 and 70; and
a primer set for amplifying the cytochrome b gene that is an oligonucleotide pair having nucleotide sequences selected from those of SEQ ID NOS: 71 to 85, and uses one or more combinations of a mixture of SEQ ID NOS: 71 to 75 and a mixture of SEQ ID NOS: 76 to 78, or a mixture of SEQ ID NOS: 79 to 82 and a mixture of SEQ ID NOS: 83 to 85.

The present invention further provides a method for determining an animal biospecies of which component is contained in a test specimen by hybridizing any of these DNA probes with one more more oligonucleotides immobilized on a substrate, and identifying an oligonucleotide derived from a gene having a completely matching nucleotide sequence and involved in determination of an animal biospecies.

When an animal biospecies is determined by using the present invention, the determination can be completed in about 6 hours after receiving a test sample.

According to the present invention, whether a tissue or cell component derived from a specific biospecies is contained in a sample such as feed, feed additive, feed-related product, processed food, processed food raw material, food additive, pharmaceutical preparation, pharmaceutical additive, functional food, cosmetic or cosmetic additive or not can be extremely accurately determined.

### Brief explanation of the Drawings

Fig. 1 shows one example of a layout of a substrate on which the oligonucleotides of the present invention are immobilized. The symbols represent position markers.
Fig. 2 (photograph) shows a result of a test in which the 12S rRNA gene of DNA extracted from beef was amplified and used as a probe according to the method of the present invention.
Fig. 3 (photograph) shows a result of a test in which the 16S rRNA gene of DNA extracted from mutton was amplified and used as a probe according to the method of the present invention.
Fig. 4 (photograph) shows a result of a test in which the 12S rRNA gene of DNA extracted from pork was amplified and used as a probe according to the method of the present invention.

### Detailed Description of the Preferred Embodiments

Hereafter, constitution of the kit of the present invention, a method for preparing the same and a method for determining an animal species will be explained.

### <1> Synthetic oligonucleotide used in the present invention

The synthetic oligonucleotide used in the present invention is an oligonucleotide synthesized based on a nucleotide sequence derived from a DNA sequence corresponding to the 12S rRNA, a DNA sequence corresponding to the 16S rRNA or a sequence corresponding to the cytochrome b. Hereafter, such an oligonucleotide is also referred to as "capture oligo".

Other synthetic oligonucleotides according to the present invention are a synthetic oligonucleotide comprising an artificial sequence (hereinafter, also referred to as "positive control oligo") and an oligonucleotide corresponding to a nucleotide sequence of the aforementioned positive control oligo including artificial substitution of nucleotides for one or more nucleotides in the range of less than 20% of the nucleotide numbers of the sequence (hereinafter, also referred to as "negative control oligo").

The oligonucleotides used in the present invention will be explained hereafter.

### (1) Design of capture oligo

An animal species is determined by hybridizing a DNA fragment having a part of a nucleotide sequence obtained by selecting one or more of DNA sequences corresponding to the 12S rRNA, DNA sequences corresponding to the 16S rRNA or DNA sequences corresponding to the cytochrome b (referred to as "DNA probe") with a capture oligo. The animal species from which the DNA fragment is derived can be determined according to the capture oligo with which the DNA fragment is hybridized. That is, the animal species can be accurately determined by observing an oligonucleotide spot on a substrate on which the DNA fragment is hybridized.

The capture oligo can be prepared as a synthetic oligonucleotide based on a sequence comprising 12 to 24 nucleotides including species-specific nucleotides in the nucleotide sequence selected from DNA sequences corresponding to the 12S rRNA, DNA sequences corresponding to the 16S rRNA or DNA sequences corresponding to the cytochrome b. The position of a mutation point in the capture oligo is not limited in design so long as the capture oligo can hybridize with the DNA probe, but it is usually preferred that it should exist in the vicinity of the center of the sequence. When the capture oligo is too short, detection of hybridization becomes difficult. When it is too long, non-specific nucleotides may also be contained, and hence inhibition of hybridization by a specific sequence may not occur. While a standard nucleotide sequence length is 12 to 24 nucleotides, optimization of this oligonucleotide length primarily depends on sequence characteristics (content of specific nucleotides and repeat of the same nucleotides). It was confirmed by experiments according to the present invention that a capture oligo having favorable binding property may have a short strand.

Examples of nucleotide sequence of the capture oligo are shown as SEQ ID NOS: 1 to 57. While the gene information was obtained from published references and databases, the design of the capture oligos was based on data analysis results obtained through testing by the inventors of the present invention themselves, and it can be made to be at a level that characteristic nucleotides occurring in each animal species can be extensively covered.

A specific animal species can be determined by selecting an oligonucleotide from these examples and using it in a test. While the standard acceptable range of the capture oligo length is 12 to 24 nucleotides, the range is narrowed to 14 to 22 nucleotides in actual practice and further to the range of 16 to 17 nucleotides as a median size in view of hybridization efficiency.

In addition to the nucleotide sequences of SEQ ID NOS: 1 to 4, 13 to 16, 24 to 27, 36 to 39 and 47 to 50, an oligonucleotide having a nucleotide sequence obtained by extending or shortening the DNA sequence corresponding to the 12S rRNA in each nucleotide sequence at the 5' or 3' end or the both of each nucleotide sequence can be used as a capture oligo. Further, in addition to the nucleotide sequences of SEQ ID NOS: 5 to 8, 17 to 20, 28 to 31, 40 to 43 and 51 to 54, an oligonucleotide having a nucleotide sequence obtained by extending or shortening the DNA sequence corresponding to the 16S rRNA in each nucleotide sequence at the 5' or 3' end or the both of each nucleotide sequence can be used as a capture oligo. Further, in addition to the nucleotide sequences of SEQ ID NOS: 9 to 12, 21 to 23, 32 to 35, 44 to 46 and 55 to 57, an oligonucleotide having a nucleotide sequence obtained by extending or shortening the DNA sequence corresponding to the cytochrome b gene sequence in each nucleotide sequence at the 5' or 3' end or the both of each nucleotide sequence can be used as a capture oligo. The nucleotide sequences of the 12S rRNA gene, 16S rRNA gene and cytochrome b gene can be obtained from the database of GenBank/EMBL/DDBJ or the like. The sequences of the extended or shortened oligonucleotides can be designed with reference to these sequences.

The capture oligos designed in the present invention reflect the results of studies and researches up to the point of the present application. However, when additional information is obtained thereafter, new capture oligos can be prepared based on the design method described in the present application, and these also fall within the scope of the present invention.

Synthesis of oligonucleotides, preparation of chromosomal DNA, hybridization and PCR described later and so forth can be performed by usual methods known to those skilled in the art (see Maniatis, T. et al., "Molecular Cloning A Laboratory Manual, Second Edition", Cold Spring Harbor Laboratory Press (1989) etc.). Further, oligonucleotides can be synthesized by using a commercially available DNA synthesizer.

When an oligonucleotide has a hairpin structure, loop structure or any other three-dimensional structure that interferes with hybridization with a test nucleic acid, the three-dimensional structure can be canceled by substituting inosine or a nucleic acid that does not pair with any nucleotide for one or more nucleotides constituting the oligonucleotide.

### (2) Design of control oligo

Whether an amplification reaction is successfully carried out or not can be determined by hybridization with a positive control oligo. Further, a negative control oligo is used to confirm that hybridization of an oligonucleotide and a DNA probe is specific, that is, a false-positive hybridization signal is not generated.

### <2> Preparation of substrate used for the present invention

Material of a substrate on which oligonucleotides are immobilized may be a material on which the oligonucleotides can be stably immobilized, and examples thereof include glass and synthetic resins such as polycarbonate and plastics. Although the shape of the substrate is not particularly limited, a plate or a film can be mentioned. A substrate having a uniform and flat surface is suitable.

For immobilization of the oligonucleotides onto the substrate, techniques used for a usual hybridization method such as physical adsorption, electrical coupling or molecular covalent bonds can be utilized. However, in the present invention, a substrate having carbodiimide groups or isocyanate groups on its surface is used (U.S. Patent No. 5,908,746; Japanese Patent Laid-open Publication (Kokai) No. 8-23975), and irradiated with an ultraviolet ray to immobilize oligonucleotides with covalent bonds. The inventors of the present invention succeeded in significantly increasing immobilization efficiency by irradiating a substrate having carbodiimide groups or isocyanate groups on its surface with an ultraviolet ray (Japanese Patent Application No. 2000-225985).

### <3> Immobilization of oligonucleotide

If the amount of spotted oligonucleotides is too small when oligonucleotides are spotted, sufficient reactivity between the oligonucleotides and a DNA probe cannot be secured, and hence determination may become difficult. Further, highly integrated spotting has problems in techniques as well as costs, and a more precise and expensive detection device (typically, a scanner) is also required to determine presence or absence of hybridization of a DNA probe by using fluorescence labeling, chemical color development or the like. Therefore, it is preferable to immobilize the oligonucleotides on the substrate surface in a size of 10 to 1000 µm in diameter. Oligonucleotides can be immobilized by, for example, spotting an oligonucleotide solution on a substrate by using a spotting machine. The oligonucleotide solution is usually spotted in a substantially circular shape.

### <4> Preparation of nucleic acid from test sample and preparation of DNA probe

A nucleic acid can be prepared from a test sample in the same manner as in a usual nucleic acid preparation method. For example, DNA can be extracted according to the method described by R-F. Wang (Molecular and Cellular Probes, 14, 1, 1-5, 2000). While this is a standard preparation method, there are many alternative methods, and any of these may be used.

A DNA probe for determination is prepared by using the extracted DNA as a raw material. The DNA probe can be prepared by DNA amplification using a primer designed so as to correspond to the nucleotide sequence of a capture oligo or a control oligo. Examples of the nucleic acid amplification method include amplification as DNA by PCR and amplification as RNA by the *in vitro* transcription method.

The primers used for PCR are designed so that the DNA probe should include a nucleotide sequence complementary to a capture oligo or a control oligo except for a nucleotide at a species-specific point. The DNA probe may be longer or shorter than a capture oligo or a control oligo so long as hybridization is possible.

In order to obtain a labeled DNA probe, the primer used for PCR can be labeled beforehand, or the DNA probe may be labeled during or after PCR. As a labeling substance, the same labeling substance as used for a probe used in usual hybridization such as a fluorescent substance or a hapten can be used. Specific examples of the fluorescent substance include fluorescein (FITC), Rhodamine, phycoerythrin (PE), Texas Red, cyanine fluorochrome and so forth. Specific examples of the hapten include biotin, digoxigenin (Dig), dinitrophenyl (DNP), fluorescein and so forth.

### <5> Hybridization of DNA probe with oligonucleotide on substrate

The hybridization can be performed in the same manner as in usual nucleic acid hybridization. A specific method will be exemplified below.

The DNA probe is added to a fusion solution comprising a salt solution such as Standard Saline Citrate (SSC), a blocking solution such as sodium dodecylsulfate (SDS) or bovine serum albumin (BSA) and additives for promoting a fusion reaction. When the probe is double-stranded, denaturation is performed by heat or the like. Several microliters of a DNA probe solution is added onto a substrate and heated (usually 37°C to 50°C) for several hours to form a hybrid between an oligonucleotide immobilized on a substrate and the DNA probe.

5 x SSC or 3 M tetramethylammonium chloride is added onto the substrate and heated (usually at 37°C to 50°C) to remove nonspecific oligonucleotides, which do not form hybrids, from the substrate and selectively leave only specific hybrids on the substrate.

A fluorescent substance or a hapten introduced into the DNA probe is used for detection of a hybrid. When a hapten is used, a solution containing a conjugate (enzyme conjugate) of a protein that recognizes or binds to a hapten and an enzyme such as alkaline phosphatase or horseradish peroxidase is added onto the substrate and allowed to react at room temperature for several tens of minutes. A nonspecific adsorption reaction between the enzyme conjugate and the substrate can be inhibited by completely blocking a region of the substrate other than the oligonucleotide-immobilized region with a protein such as casein before a binding reaction of the hapten and the enzyme conjugate is carried out. This can be attained by immobilizing oligonucleotides on the substrate, then adding a solution of a protein such as casein onto the substrate and leaving the substrate at room temperature for several tens of minutes.

After completion of the binding reaction of the enzyme conjugate and the hapten in the DNA probe, the enzyme conjugate that does not bind to the hapten is washed off with an appropriate buffer containing a surfactant. As a result, only the enzyme conjugate binding to the hapten in the DNA probe remains on the substrate.

In order to visualize the hybrid, a compound that forms an insoluble compound only when only the binding product of the hapten and the enzyme conjugate exists is added. Generation of the insoluble compound is amplified by an enzymatic reaction and thus visualized. When the enzyme in the enzyme conjugate is alkaline phosphatase, nitroblue tetrazolium chloride (NBT) and 5-bromo-4-chloro-3-indolylphosphate-p-toluidine salt (BCIP) are used as the compounds to be added. When the enzyme is horseradish peroxidase, 3,3',5,5'-tetramethylbenzidine (TMB) or the like is used.

An animal species contained in the test specimen is determined by observing pigmentation or fluorescent color development of the hybrid at a position at which capture oligos are immobilized. That is, an animal species corresponding to a position showing pigmentation or fluorescent color development is the contained animal species.

Further, when a positive signal is observed at a position of the positive control oligo, it is indicated that this test is normally functioning. When a positive signal is observed at a position of a negative control oligo, it is indicated that hybridization is not carried out under appropriate conditions.

The kit of the present invention contains a substrate on which oligonucleotides are immobilized. Further, the kit of the present invention contains reagents for hybridization such as a primer for preparing a DNA probe or a labeled DNA probe, a buffer and an enzyme conjugate that recognizes a hapten.

### Examples

Hereafter, the present invention will be explained more specifically with reference to the following examples.

### Example 1: Synthesis of oligonucleotide

Oligonucleotides having an amino group or a hydroxyl group at the 5' end were synthesized by using an oligonucleotide synthesizer (Perkin-Elmer Applied Biosystems), deprotected and dried in a conventional manner. This dried oligonucleotides were dissolved by using a buffer of 10 mM Tris-HCl (pH 7.5), 1 mM EDTA to prepare a 100 pmol/µl oligonucleotide solution. This synthesis method is similarly used for any of oligonucleotides used as a capture oligo. The nucleotide sequences of the synthesized oligonucleotides are shown as SEQ ID NOS: 1 to 92 in Sequence Listing.

### Example 2: Spotting of oligonucleotide onto substrate (case where oligonucleotide having amino group at 5' end is used)

In an amount of 10 µl of solutions of an oligonucleotide having an amino group at the 5' end was mixed with 10 µl of a microspotting solution (TeleChem International Inc.) and dividedly added onto a microtiter plate (Greiner Labotechnik Ltd.). Silanated slide glass (Matsunami Glass Ind., Ltd.) was placed at a predetermined position of a spotting machine, and the spotting machine was operated. After completion of the spotting, the slide glass was exposed to vapor from hot water for several seconds, and then irradiated with 30 mJ of an ultraviolet ray. The slide glass was exposed to vapor for several seconds again and placed on a hot plate to remove moisture. The slide glass was rinsed with 0.1% aqueous sodium dodecylsulfate and then with distilled water. The slide glass was immersed in a buffer of 100 mM Tris-HCl (pH 7.5), 100 mM NaCl, 0.1% Triton X-100 containing 3% bovine serum albumin (BSA) at room temperature for 30 minutes for blocking. Then, the slide glass was dried at room temperature and washed with a buffer of 10 mM Tris-HCl (pH 7.5), 1 mM EDTA. The slide glass was dried at room temperature again and stored in a dry state in a dark cold place before use.

### Example 3: Spotting of oligonucleotide onto substrate (case where oligonucleotide having hydroxyl group at 5' end is used)

In an amount of 10 µl of a solution of an oligonucleotide having a hydroxyl group at the 5' end was mixed with 10 µl of a spotting solution (2 M aqueous sodium chloride) and dividedly added onto a microtiter plate (Greiner Labotechnik Ltd.). Slide glass having carbodiimide groups was placed at a predetermined position of a spotting machine, and the spotting machine was operated. After completion of the spotting, the slide glass was placed in a drier at 37°C for 30 minutes. The slide glass was immersed in a buffer of 100 mM Tris-HCl (pH 7.5), 100 mM NaCl, 0.1% Triton X-100 containing 3% BSA at room temperature for 30 minutes for blocking. Then, the slide glass was dried at room temperature and washed with a buffer of 10 mM Tris-HCl (pH 7.5), 10 mM EDTA. The slide glass was dried at room temperature again and stored in a dry state in a dark cold place before use.

### Example 4: Preparation of DNA probe

According to the method described by R-F. Wang, DNA was extracted (Molecular and Cellular Probes 14, 1, 1-5, 2000).

In PCR amplification, 1 unit of Z-Taq polymerase (Takara Shuzo), 1 µl each of 100 pmol/µl 5' end primer and 3' end primer, 2 µl of X10 reaction buffer, 2 µl of 2.5 mM dNTP (Gibco BRL), 1 µl of 10 ng/µl genomic DNA and 1 µl of 1 ng/µl DNA to be added (obtained by annealing the synthetic oligonucleotides of SEQ ID NOS: 87 and 88) were added to a tube, and added with sterilized distilled water to a total volume of 20 µl. The tube was set in a thermal cycler (PTC-200, MJ Research Inc.), and a program was operated for a heating cycle consisting of reactions (1) at 98°C for 2 minutes, (2) at 98°C for 5 seconds, (3) at 55°C for 5 seconds, (4) at 72°C for 10 seconds and (5) at 72°C for 2 minutes, in which reactions (2) to (4) were repeated 50 times.

In this example, electrophoresis using an agarose gel described below was performed as a verification test. However, it is unnecessary in actual identification.

After completion of the probe synthesis reaction, 1 µl was taken from the reaction solution and mixed with 1 µl of 6X electrophoretic pigment (30% glycerol, 0.25% bromophenol blue, 0.25% xylene cyanol) and 4 µl of a buffer of 10 mM Tris-HCl (pH 7.5), 1 mM EDTA. After the mixing, the mixture was subjected to electrophoresis on a 2% agarose gel at 100 V for 25 minutes. After the electrophoresis, the gel was immersed in distilled water containing 0.5 µg/ml of ethidium bromide for 15 minutes and irradiated with an ultraviolet ray to record the result as a photograph.

### Example 5: Hybridization

In an amount of 5 µl of the DNA probe prepared in Example 4 was added to a 1.5 ml-volume tube, further added with 20 µl a UniHyb Hybridization Solution (TeleChem International Inc.) and mixed to prepare a hybridization solution. This solution was heated at 100°C for 10 minutes and immersed in ice for 5 minutes. In an amount of 10 µl of this DNA probe solution was placed on the substrates prepared in Examples 2 and 3, on which oligonucleotides were spotted, and cover glass was placed thereon. Each of the substrates was loaded into a hybridization cassette device (TeleChem International Inc.), placed on a water bath at 42°C and left for 1 hour with light shielding. The substrate was removed from the hybridization cassette device and immersed in 5 x SSC (0.75 M NaCl, 0.075 M sodium citrate) at 4°C to remove the cover glass. The operation of immersing the substrate in 5 x SSC at 4°C for 30 minutes was repeated twice, and the substrate was rinsed twice with 3 M aqueous tetramethylammonium chloride at room temperature and then twice with 3 M aqueous tetramethylammonium chloride at 45°C. Finally, the substrate was transferred to a vessel containing 2 x SSC.

### Example 6: Detection of chemical color development

The substrate was removed from the vessel, and moisture in portions on which oligonucleotides were not immobilized was removed with paper towel, while 200 µl of BlockAce solution (Dainippon Pharmaceutical) was placed on portions on which oligonucleotides were immobilized. The substrate was left at room temperature for 30 minutes, and the solution was sucked by a dispenser. In an amount of 5 ml of a TBST solution (50 mM Tris-HCl (pH 7.6), 0.15 M NaCl, 0.05% Tween 20) was added with 1 drop each of avidin DH and biotinylated peroxidase (Vector Laboratories, Inc.) and mixed, 200 µl of the mixed solution was placed on the nucleotide-immobilized portions of the substrate, and the substrate was left at room temperature for 30 minutes. The solution was sucked by the dispenser, and the substrate was transferred to a vessel containing a TBST solution and washed at room temperature for 5 minutes while allowing penetration of the solution. The solution was once discarded, a fresh TBST solution was added to the vessel, and the substrate was washed at room temperature for 5 minutes while allowing penetration of the solution. The substrate was removed from the vessel, and moisture on the portions on which oligonucleotides were not immobilized was removed with paper towel. In an amount of 200 µl of TMB solution (Vector Laboratories, Inc.) was placed on the oligonucleotide-immobilized regions and allowed to react at room temperature for 20 minutes, and the substrate was washed with deionized water to terminate the enzymatic reaction.

### Example 7: Determination

After the detection of chemical color development, the result was stored in a computer as a TIFF image by using an OA scanner (NEC Multireader 600SR) and Photoshop Ver. 5.0 (Adobe Systems Inc.). Based on this image, when pigmentation was observed in the chemical color development detection, it was indicated that the oligonucleotide (capture oligo) immobilized on the substrate and the DNA probe in the specimen were completely complementary to each other and formed double strands. When the size of spotted capture oligo is large, this can be detected with a magnifying glass.

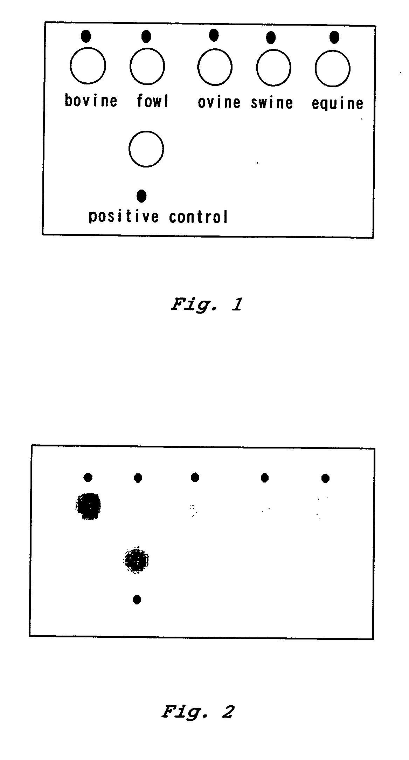

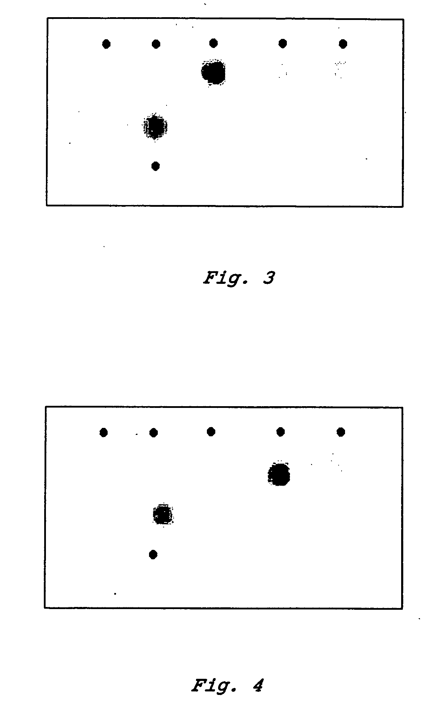

Fig. 1 shows a layout of the array. As examples, Figs. 2 to 4 show the test results obtained when the 12S rRNA genes of DNA extracted from beef, mutton and pork, respectively, were amplified and used as the probes.
The sequence numbers of the used capture oligos and primers are shown in Table 1.

**Table 1**

| | SEQ ID NOS: |
|---|---|
| Primer for amplification of 5' side | Mixture of equal amounts of 58, 59, 60 |
| Primer for amplification of 3' side | Mixture of equal amounts of 61, 62 |
| Capture for bovine 12S | 1 |
| Capture for fowl 12S | 13 |
| Capture for ovine 12S | 24 |
| Capture for swine 12S | 36 |
| Capture for equine 12S | 47 |
| Positive control capture | 86 |
| DNA to be added | 87, 88 |

## Claims

1. A method for determining presence or absence of a component derived from an organism contained in a test material or a biospecies from which the component is derived, which comprises detecting a nucleic acid specific to a biospecies of interest by performing hybridization of an oligonucleotide synthesized based on a sequence specific to the biospecies in a DNA sequence corresponding to a ribosomal RNA gene or a cytochrome-related gene of the biospecies of interest and a nucleic acid derived from the test material.

2. The method according to claim 1, wherein the biospecies is one or more kinds selected from the group consisting to bovine, ovine, swine, equine and fowl.

3. The method according to claim 1, wherein the test material is selected from the group consisting of feed, feed raw material, feed additive, feed-related product, processed food, processed food raw material, food additive, pharmaceutical preparation, pharmaceutical additive, functional food, cosmetic or cosmetic additive.

4. The method according to any one of claims 1 to 3, wherein the DNA sequence corresponding to a ribosomal RNA gene is a DNA sequence corresponding to the 12S ribosomal RNA gene or the 16S ribosomal RNA gene, and the DNA sequence corresponding to a cytochrome-related gene is a DNA sequence corresponding to the cytochrome b gene.

5. The method according to any one of claims 1 to 4, wherein the biospecies is bovine, the DNA sequence corresponding to the 12S ribosomal RNA gene contains any of the nucleotide sequences of SEQ ID NOS: 1 to 4, the DNA sequence corresponding to the 16S claim gene contains any of the nucleotide sequences of SEQ ID NOS: 5 to 8, and the DNA sequence corresponding to the cytochrome b gene contains any of the nucleotide sequences of SEQ ID NOS: 9 to 12.

6. The method according to any one of claims 1 to 4, wherein the biospecies is fowl, the DNA sequence corresponding to the 12S ribosomal RNA gene contains any of the nucleotide sequences of SEQ ID NOS: 13 to 16, the DNA sequence corresponding to the 16S ribosomal RNA gene contains any of the nucleotide sequences of SEQ ID NOS: 17 to 20, and the DNA sequence corresponding to the cytochrome b gene contains any of the nucleotide sequences of SEQ ID NOS: 21 to 23.

7. The method according to any one of claims 1 to 4, wherein the biospecies is ovine, the DNA sequence corresponding to the 12S ribosomal RNA gene contains any of the nucleotide sequences of SEQ ID NOS: 24 to 27, the DNA sequence corresponding to the 16S ribosomal RNA gene contains any of the nucleotide sequences of SEQ ID NOS: 28 to 31, and the DNA sequence corresponding to the cytochrome b gene contains any of the nucleotide sequences of SEQ ID NOS: 32 to 35.

8. The method according to any one of claims 1 to 4, wherein the biospecies is swine, the DNA sequence corresponding to the 12S ribosomal RNA gene contains any of the nucleotide sequences of SEQ ID NOS: 36 to 39, the DNA sequence corresponding to the 16S ribosomal RNA gene contains any of the nucleotide sequences of SEQ ID NOS: 40 to 43, and the DNA sequence corresponding to the cytochrome b gene contains any of the nucleotide sequences of SEQ ID NOS: 44 to 46.

9. The method according to any one of claims 1 to 4, wherein the biospecies is equine, the DNA sequence corresponding to the 12S ribosomal RNA gene contains any of the nucleotide sequences of SEQ ID NOS: 47 to 50, the DNA sequence corresponding to the 16S ribosomal RNA gene contains any of the nucleotide sequences of SEQ ID NOS: 51 to 54, and the DNA sequence corresponding to the cytochrome b gene contains any of the nucleotide sequences of SEQ ID NOS: 55 to 57.

10. A kit for determining presence or absence of a component derived from an organism contained in a test material or a biospecies from which the component is derived, which comprises:
a substrate on which one or more oligonucleotides synthesized based on a sequence specific to a biospecies of interest in a DNA sequence corresponding to a ribosomal RNA gene or a cytochrome-related gene of the biospecies are immobilized with a covalent bond, wherein
a nucleic acid specific to the biospecies of interest is detected by hybridization of the oligonucleotide and a nucleic acid derived from the test material.

11. The kit according to claim 10, wherein a carbodiimide group or an isocyanate group is provided on the surface of the substrate so that the covalent bond should be formed by a reaction of the carbodiimide group or isocyanate group and the oligonucleotide or a linker added to an end of the oligonucleotide.

12. The kit according to claim 10, wherein the biospecies is one or more kinds selected from the group consisting of bovine, ovine, swine, equine and fowl.

13. The kit according to claim 10, wherein the test material is selected from the group consisting of feed, feed raw material, feed additive, feed-related product, processed food, processed food raw material, food additive, pharmaceutical preparation, pharmaceutical additive, functional food, cosmetic or cosmetic additive.

14. The kit according to any one of claims 10 to 13, wherein the DNA sequence corresponding to a ribosomal RNA gene is a DNA sequence corresponding to the 12S ribosomal RNA gene or the 16S ribosomal RNA gene, and the DNA sequence corresponding to a cytochrome-related gene is a DNA sequence corresponding to the cytochrome b gene.

15. The kit according to any one of claims 10 to 14, wherein the biospecies is bovine, the DNA sequence corresponding to the 12S ribosomal RNA gene contains any of the nucleotide sequences of SEQ ID NOS: 1 to 4, the DNA sequence corresponding to the 16S ribosomal RNA gene contains any of the nucleotide sequences of SEQ ID NOS: 5 to 8, and the DNA sequence corresponding to the cytochrome b gene contains any of the nucleotide sequences of SEQ ID NOS: 9 to 12.

16. The kit according to any of claims 10 to 14, wherein the biospecies is fowl, the DNA sequence corresponding to the 12S ribosomal RNA gene contains any of the nucleotide sequences of SEQ ID NOS: 13 to 16, the DNA sequence corresponding to the 16S ribosomal RNA gene contains any of the nucleotide sequences of SEQ ID NOS: 17 to 20, and the DNA sequence corresponding to the cytochrome b gene contains any of the nucleotide sequences of SEQ ID NOS: 21 to 23.

17. The kit according to any of claims 10 to 14, wherein the biospecies is ovine, the DNA sequence corresponding to the 12S ribosomal RNA gene contains any of the nucleotide sequences of SEQ ID NOS: 24 to 27, the DNA sequence corresponding to the 16S ribosomal RNA gene contains any of the nucleotide sequences of SEQ ID NOS: 28 to 31, and the DNA sequence corresponding to the cytochrome b gene contains any of the nucleotide sequences of SEQ ID NOS: 32 to 35.

18. The kit according to any of claims 10 to 14, wherein the biospecies is swine, the DNA sequence corresponding to the 12S ribosomal RNA gene contains any of the nucleotide sequences of SEQ ID NOS: 36 to 39, the DNA sequence corresponding to the 16S ribosomal RNA gene contains any of the nucleotide sequences of SEQ ID NOS: 40 to 43, and the DNA sequence corresponding to the cytochrome b gene contains any of the nucleotide sequences of SEQ ID NOS: 44 to 46.

19. The kit according to any of claims 10 to 14, wherein the biospecies is equine, the DNA sequence corresponding to the 12S ribosomal RNA gene contains any of the nucleotide sequences of SEQ ID NOS: 47 to 50, the DNA sequence corresponding to the 16S ribosomal RNA gene contains any of the nucleotide sequences of SEQ ID NOS: 51 to 54, and the DNA sequence corresponding to the cytochrome b gene contains any of the nucleotide sequences of SEQ ID NOS: 55 to 57.

20. A method for producing a labeled DNA probe derived from DNA of a test material used for identifying a biospecies by using the method according to claim 1, comprising the steps of:
extracting DNA from the test material, and
performing DNA amplification by using the DNA as a template and a primer of which 5' or 3' end is labeled with a fluorescent substance or a hapten.

21. A DNA probe obtainable by the method according to claim 20, which is labeled with any of fluorescent substances including fluorescein (FITC), Rhodamine, phycoerythrin (PE), Texas Red and cyanine fluorochrome.

22. A DNA probe obtainable by the method according to claim 20, which is labeled with any of haptens including biotin, digoxigenin (Dig), dinitrophenyl (DNP) and fluorescein.

23. A method for producing a labeled DNA probe derived from DNA of a test material used for identifying a biospecies by using the kit according to claim 10, comprising the steps of:
extracting DNA from the test material, and
performing DNA amplification by using the DNA as a template and a primer of which 5' or 3' end is labeled with a fluorescent substance or a hapten.

24. A primer for producing a nucleic acid derived from DNA of a test material used in the method according to claim 1, which has a sequence corresponding to the 12S ribosomal RNA gene of any one or more kinds of animals selected from the group consisting of bovine, ovine, equine, swine and fowl and has a nucleotide sequence selected from the nucleotide sequences of SEQ ID NOS: 58 to 64.

25. A primer for producing a nucleic acid derived from DNA of a test material used in the method according to claim 1, which has a sequence corresponding to the 16S ribosomal RNA gene of any one or more kinds of animals selected from the group consisting of bovine, ovine, equine, swine and fowl and has a nucleotide sequence selected from the nucleotide sequences of SEQ ID NOS: 65 to 70.

26. A primer for producing a nucleic acid derived from DNA of a test material used in the method according to claim 1, which has a sequence corresponding to the cytochrome b gene of any one or more kinds of animals selected from the group consisting of bovine, ovine, equine, swine and fowl and has a nucleotide sequence selected from the nucleotide sequences of SEQ ID NOS: 71 to 85.

27. The method according to any one of claims 1 to 9, wherein, when the oligonucleotide has a hairpin structure, loop structure or another three-dimensional structure that interferes with hybridization with the test nucleic acid, there is used an oligonucleotide corresponding to the oligonucleotide of which three-dimensional structure is canceled by substitution of inosine or a nucleic acid that does not pair with any nucleotide for one or more nucleotides constituting the oligonucleotide.

28. The kit according to claims 10 to 19, wherein, when the oligonucleotide has a hairpin structure, loop structure or another three-dimensional structure that interferes with hybridization with the test nucleic acid, there is used an oligonucleotide corresponding to the oligonucleotide of which three-dimensional structure is canceled by substitution of inosine or a nucleic acid that does not pair with any nucleotide for one or more nucleotides constituting the oligonucleotide.

29. The kit according to claim 11, wherein the reaction of the carbodiimide group or isocyanate group on the substrate and the oligonucleotide is carried out under ultraviolet ray irradiation.
